# FASCICULE DE BREVET EUROPEEN

(11) **EP 1 606 198 B1**
(45) Date de publication et mention de la délivrance du brevet: **05.01.2011**
(21) Numéro de dépôt: 04742357.9
(22) Date de dépôt: 25.03.2004
(51) Int. Cl.: B65D 85/50

(54) **UTILISATION d'UN CONDITIONNEMENT POUR LA LUTTE BIOLOGIQUE CONTRE LES RAVAGEURS DES PLANTES**
VERWENDUNG VON BEHÄLTER ZUR BIOLOGISCHEN BEKÄMPFUNG
USE OF PACKAGING IN THE BIOLOGICAL ERADICATION OF PLANT RAVAGERS

(30) Priorité: 26.03.2003 FR 0303723; 10.06.2003 FR 0306945
(43) Date de publication de la demande: 21.12.2005
(73) Titulaire: Biotop, 75782 Paris Cédex 16 (FR)
(72) Inventeur: FRANDON, Jacques, F-06600 Antibes (FR); KABIRI, Firouz, F-06100 Nice (FR)
(74) Mandataire: Ahner, Francis
(86) Numéro de dépôt international: PCT/FR2004/000752
(87) Numéro de publication internationale: WO 2004/087536

(56) Documents cités:
- FR-A- 2 746 259
- GB-A- 2 211 717
- US-A- 3 375 808
- US-A- 3 480 197
- US-A- 5 686 127
- US-A1- 2002 008 105

## Description

Pour utiliser avec efficacité les auxiliaires destinés à la lutte biologique contre les ravageurs des plantes, il faut disposer d'un conditionnement permettant de les protéger. Cette protection doit être efficace, d'une part, contre les prédateurs et, d'autre part, contre les intempéries en milieu extérieur (notamment la pluie et l'irrigation par aspersion).

La présente invention concerne donc l'utilisation d'un conditionnement permettant la protection et la libération d'insectes et/ou d'acariens auxiliaires utilisables en lutte biologique contre les ravageurs des plantes. Ce conditionnement est remarquable dans son utilisation par le fait qu'il est adaptable à tout type d'insectes et d'acariens auxiliaires. La présente invention concerne, plus particulièrement, l'utilisation d'un conditionnement permettant la protection et la libération des Trichogrammes, parasitoïdes d'oeufs de lépidoptères, et d'autres auxiliaires. On décrit également dans la présente le procédé de fabrication dudit conditionnement et son utilisation en lutte biologique.

Le Trichogramme est un insecte micro-hyménoptère dont l'adulte mesure environ 0,5 mm. Il est utilisé en lutte biologique depuis des dizaines d'années, pour protéger les cultures des attaques des chenilles de lépidoptères ravageurs.

Cet auxiliaire est, en général, conditionné et lâché sur les cultures à protéger, sous forme d'oeufs de papillon parasités. L'insecte poursuit son développement dans l'oeuf de papillon avant d'émerger et d'aller à la recherche des oeufs du lépidoptère ravageur visé. Il faut donc avoir une protection pour toute la durée entre le lâcher de l'auxiliaire et son émergence. Il existe différentes méthodes d'utilisation dont l'efficacité et le coût dépendent étroitement de la qualité du système de protection de l'auxiliaire.

Certaines méthodes d'utilisation sont très simples mais aussi peu efficaces : les oeufs parasités sont épandus, juste avant l'émergence des trichogrammes, en vrac ou collés sur un support donné sans protection. Dans ce cas, un grand nombre d'auxiliaires sont perdus car, sans protection, ils sont la proie de différents prédateurs. De telles méthodes entraînent également des pertes importantes en cas d'intempéries.

D'autres méthodes envisagent de protéger les oeufs parasités. En effet, lesdits oeufs se trouvent sur des supports plus ou moins bien fermés. Les protections les plus performantes sont aussi les plus difficiles ou les plus coûteuses à réaliser.

L'état de la technique connaît également un procédé de production en masse de trichogrammes, ce procédé fait l'objet du brevet européen délivré le 27 juin 1990 sous le numéro EP0213405. Dans ce document, les capsules dans lesquelles les insectes produits sont conditionnés en vue de leur utilisation en lutte biologique sont des capsules en carton-pâte de 1,2 cm de diamètre. Ces capsules se présentent sous la forme de deux demi-sphères collées puis ultérieurement percées de trous de sortie avant le lâcher au champ. Le perçage de ces capsules tel que décrit dans ce document est réalisé au moyen d'un dispositif à aiguilles, soit juste avant le lâcher au champ, soit juste après le stockage des capsules. Dans les deux cas, le perçage est effectué alors que les insectes sont déjà dans la capsule.

Les capsules proposées jusqu'à présent sont obtenues après plusieurs opérations qui sont :
- le collage de plaques alvéolées,
- la mise en place des oeufs dans les alvéoles,
- la mise en place d'une plaque alvéolée vide sur la première puis
- le pressage et la découpe pour obtenir des capsules entièrement fermées.

Ces capsules sont ensuite reprises par un autre équipement pour être percées, avant d'être comptées pour définir les unités de vente destinées aux utilisateurs.

La reprise des capsules pour le perçage entraîne un surcoût relativement important du fait du temps et des manutentions supplémentaires, mais aussi du fait qu'au cours de ces opérations supplémentaires, un certain nombre de capsules sont détériorées et se trouvent donc non commercialisables.

Le document US 2002/0008105A1 divulgue l'utilisation d' un conditionnement pour protéger et libérer des insectes dans le cadre de la lutte biologique, le conditionnement comprenant un récipient muni d'une ouverture.

La présente invention vise donc à résoudre les problèmes cités ci-dessus et propose un conditionnement présentant des orifices de sortie réalisés dès la fabrication dudit conditionnement sans avoir à le reprendre pour perçage. Le conditionnement objet de la présente invention est également remarquable de par le fait qu'il permet d'obtenir une très bonne protection des auxiliaires qu'il contient. En effet, la dimension des orifices de sortie et leur nombre sont définis en fonction de la taille et du nombre des auxiliaires.

La présente invention vise donc à résoudre les problèmes cités ci-dessus et est définie par l'utilisation d'un conditionnement selon la revendication 1, le conditionnement présentant des orifices de sortie réalisés dès la fabrication dudit conditionnement sans avoir à le reprendre pour perçage. Le conditionnement est également remarquable de par le fait qu'il permet d'obtenir une très bonne protection des auxiliaires qu'il contient. En effet, la dimension des orifices de sortie et leur nombre sont définis en fonction de la taille et du nombre des auxiliaires.

Le conditionnement pour insectes et/ou acariens est constitué par
- un récipient présentant au moins une languette, elle-même présentant au moins une rainure et
- un moyen pour fermer ledit récipient, où
lorsque ledit moyen de fermeture est disposé sur ledit récipient, ladite rainure et ledit moyen pour fermer ledit récipient forment un orifice permettant la sortie desdits insectes et/ou acariens.

Par "récipient", on entend dans le cadre de la présente invention toute structure creuse dans laquelle les insectes et/ou acariens peuvent être disposés, à titre d'exemple, sous forme d'oeufs parasités. Ledit récipient peut, avantageusement, se présenter sous la forme d'une demi-sphère, d'une capsule, d'une boîte ou d'une alvéole. La taille dudit récipient caractérisée par sa surface et sa profondeur est adaptable en fonction de l'insecte et/ou de l'acarien qu'il contient (taille et nombre). A titre d'exemple et de façon non-exhaustive, un récipient destiné à conditionner des trichogrammes doit avantageusement présenter une surface de 1 cm² pour environ 400 insectes ou oeufs parasités. Le tableau 1 ci-dessous présente les caractéristiques d'un récipient de forme alvéolaire (volume utile) en tenant compte de la taille des auxiliaires. Les auxiliaires envisagés sont des trichogrammes, des acariens, des punaises prédatrices du type Orius, Anthocoris et Macrolophus et des parasites de pucerons.

**Tableau 1 : Dimensions indicatives des alvéoles (volume utile) en tenant compte de la taille des auxiliaires.**

| Auxiliaire (Nombre d'auxiliaires) | Surface du récipient (cm²) | Profondeur du récipient (mm) | Dimensions de l'orifice de sortie (mm) |
|---|---|---|---|
| Trichogrammes (1000 à 2000) | 2 à 30 | 2,2 | 1 |
| Acariens (500 à 3000) | 15 à 40 | 3 | 1 |
| Orius et Anthocoris (50 à 200) | 50 à 100 | 10 | 4x2 |
| Macrolophus (50 à 200) | 50 à 100 | 15 | 5x3 |
| Parasites de pucerons (50 à 200) | 50 à 100 | 10 | 4x2 |

De préférence, le conditionnement comprend plusieurs languettes présentant chacune au moins une rainure. Ces languettes se trouvent avantageusement sur un même plan. De façon plus particulière, ces languettes forment une bande périphérique à la surface du récipient. Ladite bande périphérique présente, de préférence, une largeur de 5 mm.

Tout moyen permettant la fermeture dudit récipient est utilisable dans le cadre de la présente invention. La seule condition est que le moyen de fermeture n'obstrue pas la (ou les) rainure(s) présente(s) sur la ou les languettes du récipient de façon à ce que, lorsque le moyen de fermeture est disposé sur celui-ci, ladite (ou lesdites) rainure(s) forme(nt) un orifice, appelé ci-après orifice de sortie. Ainsi, le moyen de fermeture du récipient peut être choisi parmi un deuxième récipient ou une surface plane telle qu'une feuille ou une plaque plate. L'homme du métier choisira le moyen de fermeture adéquat en fonction du volume intérieur recherché.

De plus, en fonction des besoins en oxygène des auxiliaires conditionnés, le moyen de fermeture dudit récipient peut comporter des micro-perforations permettant l'échange d'air.

La fermeture du récipient par le moyen de fermeture peut être réalisée par tout moyen permettant de solidariser ledit récipient avec ledit moyen de fermeture. Cette solidarisation doit avantageusement être capable de se maintenir quelles que soient les conditions climatiques que subit le conditionnement. A titre d'exemple et de façon non exhaustive, la fermeture du récipient par le moyen de fermeture peut être réalisée par collage et/ou par soudure thermique.

Par conséquent, l'orifice de sortie des insectes dans le conditionnement est formé grâce à au moins une rainure aménagée sur au moins une languette se trouvant au bord du récipient lors de la fabrication de celui-ci. Dans le cas d'un conditionnement pour trichogrammes, cette rainure a un rayon de 1 mm, comme indiqué dans le tableau 1 ci-dessus. Cette rainure permet, à la fermeture par une plaque plate, d'obtenir un orifice en demi-cercle, autorisant la sortie des Trichogrammes mais empêchant l'entrée de tout prédateur de taille supérieure à l'intérieur du conditionnement. Un même orifice permet la sortie échelonnée de plusieurs centaines d'insectes et le nombre total d'orifices peut être fixé en fonction des besoins, c'est-à-dire en fonction du nombre d'insectes conditionnés. En général, on préférera un conditionnement présentant au moins deux orifices dont un est en sécurité, au cas où l'autre viendrait à se boucher. Pour des capsules contenant plusieurs milliers d'insectes, il faut prévoir plusieurs orifices, avantageusement de 2 à 10.

Ainsi, l'adaptation de la dimension de l'orifice aux besoins exacts de l'auxiliaire conditionné permet d'optimiser la protection, aussi bien contre les prédateurs que contre les intempéries, en particulier contre l'entrée de l'eau de pluie (ou de l'irrigation par aspersion) dans le conditionnement. Cela permet une protection optimale et de longue durée. Il est ainsi possible de conditionner des auxiliaires au tout début de leur cycle ; ils pourront y terminer leur développement en toute sécurité avant d'en sortir. De ce fait, il est aussi possible de conditionner des auxiliaires à différents stades de développement. Cette possibilité permet, avec une seule capsule, de libérer des auxiliaires sur une plus longue période, simplifiant ainsi le travail des utilisateurs, dans le cas où il faudrait renouveler les apports.

Le matériau utilisable pour réaliser le conditionnement et, plus particulièrement, le récipient, la (ou les) languette (s) et le moyen de fermeture dudit récipient est choisi parmi le carton, la cellulose moulée et le plastique. Le récipient, la (ou les) languette(s) et le moyen de fermeture dudit récipient peuvent être réalisés dans un matériau identique ou différent. Le carton permet de réaliser un récipient de profondeur limitée, suffisante pour les auxiliaires de très petite taille comme les Trichogrammes. Un récipient tel qu'une alvéole avec une profondeur de 2,2 mm est tout à fait adapté pour ces auxiliaires. Pour des auxiliaires de très grande taille, il est possible d'utiliser deux récipients du type alvéole collés l'un à l'autre, le second récipient correspondant au moyen de fermeture du premier. L'utilisation d'un matériau tel que le plastique permet d'obtenir des profondeurs très importantes. Le carton présente l'avantage d'être très rapidement biodégradable et relativement peu onéreux, on peut également envisager d'utiliser des plastiques biodégradables.

Le conditionnement peut également comprendre un moyen pour l'accrocher tout particulièrement aux plantes. Tout moyen permettant l'accrochage dudit conditionnement est utilisable dans le cadre de la présente invention. Le moyen d'accrochage peut être un crochet fermé ou un crochet ouvert. Ce système d'accrochage peut être soit fixé au conditionnement après la fabrication de ce dernier, soit réalisé, au moment de la fabrication de ce conditionnement et, plus particulièrement, au moment de sa découpe, directement dans le matériau où sont moulés le récipient, le moyen de fermeture et/ou la (ou les) languette(s). Dans la seconde forme de mise en oeuvre citée ci-dessus, les deux parties du conditionnement que sont le

récipient également appelé "fond" et le moyen de fermeture dudit récipient également appelé "couvercle", sont fixées l'une à l'autre par contre-collage, ce qui permet d'obtenir une très bonne rigidité du système d'accrochage. L'homme du métier est capable de définir les dimensions du système d'accrochage en fonction de l'utilisation du conditionnement (fixation sur une tige, sur une feuille, à une branche...) .

De la nourriture peut être avantageusement disposée dans le récipient, partie du conditionnement, objet de la présente invention. Cette nourriture peut se présenter sous la forme d'une goutte de miel ou d'un grain de pollen. L'homme du métier connaît les différentes espèces d'insectes et d'acariens qui peuvent être placées dans le conditionnement et connaît, en fonction de l'espèce, la nourriture la plus adaptée à disposer dans ledit conditionnement. De plus, de par la forme du conditionnement et notamment de par la taille des orifices, la nourriture peut être disposée dans ledit conditionnement pour les auxiliaires sans que celle-ci n'attire des insectes extérieurs qui voudraient la consommer. La présence de nourriture dans le conditionnement permet d'assurer la survie des auxiliaires et, notamment des formes mobiles de ces auxiliaires et également d'améliorer leurs caractéristiques biologiques telles que leur longévité, leur fécondité,... .

Dans l'utilisation de la présente invention on peut avoir une plaque comprenant au moins deux conditionnements tels que définis ci-dessus. Une caractéristique très intéressante de la présente invention réside dans la possibilité d'utiliser des conditionnements jumelés, sécables, qui ont été réalisés en ne découpant pas complètement le pourtour du récipient, mais en laissant des points d'attache au niveau des futurs orifices de sortie. Les conditionnements sont alors séparés au moment de l'utilisation.

En effet, lorsque les conditionnements se trouvent entourés d'une bande périphérique d'une largeur de 5 mm, en laissant des points non découpés, au niveau des futurs orifices, les différents conditionnements se trouvant sur une même plaque restent attachés ensemble. Les orifices formés par les rainures au niveau de la bande périphérique du récipient restent fermés et les insectes ne peuvent pas sortir tant que les conditionnements ne sont pas séparés. Dans une telle forme de mise en oeuvre, la longueur des rainures est choisie de façon à légèrement dépasser la largeur de 5 mm de la bande périphérique autour du récipient de manière à ce que l'orifice de sortie soit bien ouvert lors de la découpe des conditionnements. La rainure, tout comme le récipient, se retrouvent dans les loges prévues à cet effet dans la matrice de l'outil de découpe, ce qui permet d'éviter leur écrasement, lors de la découpe.

Une telle possibilité est particulièrement intéressante pour lâcher des insectes mobiles, larves ou adultes, qui ne peuvent pas s'échapper tant que les conditionnements ne sont pas détachés les uns des autres. Par conséquent, ces formes mobiles ne peuvent sortir des conditionnements pendant le transport et les manutentions desdits conditionnements. Au besoin, l'utilisateur peut donc libérer immédiatement les formes mobiles des insectes et/ou acariens auxiliaires. Cette possibilité est particulièrement intéressante pour les punaises prédatrices du type Orius, Anthocoris, Macrolophus, ainsi que pour les acariens prédateurs tels que les Phytoseilus, Amblyseilus ou encore les hyménoptères parasites de pucerons tels que Aphidius, Aphelinus.

Les plaques de conditionnements permettent également de constituer des lots de conditionnements nécessaires à la protection d'une surface donnée, sans avoir à faire un conditionnement ultérieur. Il est possible de faire des plaques de 2 à 100 et, de préférence, de 20, de 30 ou de 40 conditionnements. L'homme du métier saura adapter le nombre de conditionnements par plaque en fonction de l'unité de la surface visée et en fonction de la taille des conditionnements.

La présente invention concerne l'utilisation d'un conditionnement ou d'une plaque de conditionnements tels que définis ci-dessus pour protéger et libérer des insectes et/ou acariens auxiliaires dans le cadre de la lutte biologique. Un autre avantage de la présente invention est le fait que, à partir d'un même conditionnement, les insectes et/ou acariens auxiliaires peuvent être libérés sur une longue période et ce, à différents stades de leur développement larvaire ou adulte. En effet, la présence de nourriture dans le conditionnement objet de la présente invention permet d'obtenir des auxiliaires ayant atteint leur stade adulte. Les insectes et/ou acariens auxiliaires libérés sont des formes mobiles adultes ou larvaires, alors que les insectes et/ou acariens auxiliaires sont conditionnés sous forme mobile ou non. Par conséquent, la présente invention concerne l'utilisation d'un conditionnement ou d'une plaque de conditionnements tels que définis ci-dessus pour conditionner des insectes et/ou acariens auxiliaires sous forme mobile ou non et les libérer au moment de leur utilisation sous forme mobile larvaire ou adulte. Les insectes et/ou acariens auxiliaires sont avantageusement choisis dans le groupe constitué par les trichogrammes, les punaises prédatrices du type Orius, Anthocoris, Macrolophus, ainsi que pour les acariens prédateurs tels que les Phytoseilus, Amblyseilus ou encore les hyménoptères parasites de pucerons tels que Aphidius, Aphelinus ou tout autre auxiliaire similaire.

On définit également un procédé de fabrication d'un conditionnement ou d'une plaque de conditionnements tels que définis ci-dessus , comprenant étapes suivantes et qui ne fait pas partie de l'invention:
i) le moulage d'un récipient avec au moins une languette présentant au moins une rainure,
ii) le pressage et la fermeture dudit récipient par un moyen de fermeture, et
iii) éventuellement, la découpe du conditionnement ainsi obtenu.

Le récipient, la (ou les) languette(s), le moyen de fermeture sont tels que définis précédemment. Ce procédé de fabrication permet de fabriquer des conditionnements desquels les auxiliaires sortiront ultérieurement, grâce aux orifices de sortie aménagés dès la fermeture des conditionnements.

De façon avantageuse, les auxiliaires et la nourriture peuvent être disposés dans le récipient entre l'étape (i) et l'étape (ii). Les auxiliaires peuvent être produits par toute technique de production connue de l'homme du métier. A titre d'exemple et de façon non exhaustive, la technique de production utilisable peut être celle décrite dans le brevet EP0213405.

D'autres avantages et caractéristiques de la présente invention apparaîtront à la lecture des exemples ci-après présentant deux types de conditionnement et différentes formes de mise en oeuvre des plaques de conditionnements Ces exemples qui ne sauraient être interprétés comme limitant la portée de la présente invention font référence aux figures en annexe dans lesquelles :
- la figure 1 présente une vue de dessus en perspective d'un conditionnement dans lequel le récipient et le moyen de fermeture sont encore dissociés (Figure 1A) et une vue en coupe dudit conditionnement après fermeture du récipient par le moyen de fermeture (Figure 1B).
- la figure 2 présente une vue schématique d'une plaque de conditionnements comportant deux séries de quatre conditionnements sécables munis, chacun, d'un dispositif d'accrochage aux plantes.
- la figure 3 présente une vue schématique d'une plaque de conditionnements comportant neuf conditionnements unitaires sans dispositif d'accrochage aux plantes.
- la figure 4 présente une autre forme de conditionnement. La figure 4A présente une vue de dessus en perspective du conditionnement en absence de moyen de fermeture. La figure 4B présente une vue de la base (vue A) dudit conditionnement après fermeture par le moyen de fermeture et la figure 4C une vue de côté (vue B) de ce conditionnement fermé.
- la figure 5 présente une vue schématique d'une plaque de conditionnements comportant 30 conditionnements identiques au conditionnement de la figure 4.

### I. Exemple d'un conditionnement utilisable dans la présente invention.

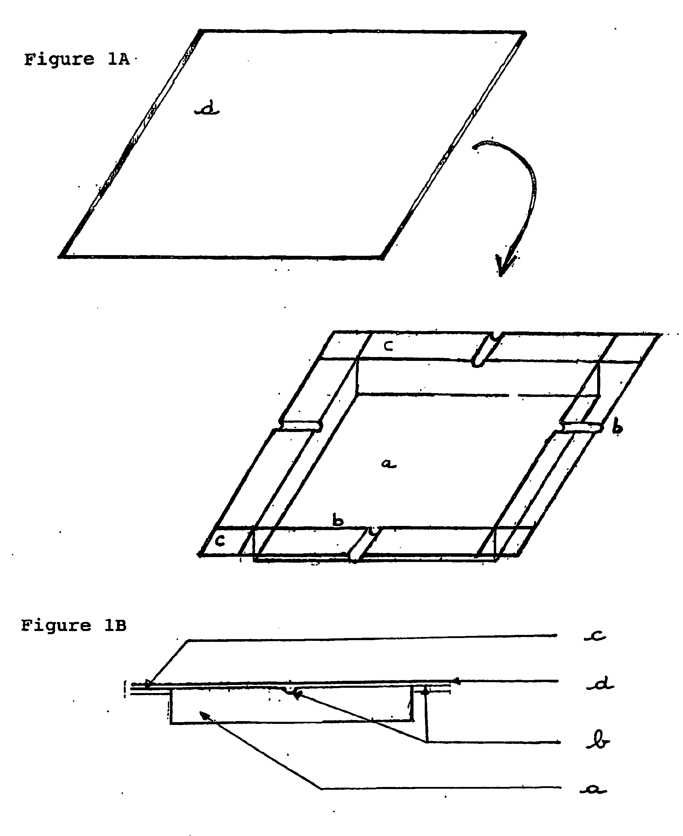

La figure 1A présente un conditionnement tel que défini dans la présente. Ce conditionnement est formé par un récipient de forme parallélépipédique (a) de type alvéolaire formant le "fond" dudit conditionnement. Ce récipient lorsque le conditionnement est utilisé pour des trichogrammes peut avantageusement présenter une hauteur de 2,2 mm avec une surface totale (parois latérales et base du récipient) comprise, de préférence, entre 2 et 30 cm². Les languettes telles que définies ci-dessous présentent la forme d'une bande périphérique (c) d'une largeur d'environ 5 mm autour du bord supérieur dudit récipient. Cette bande périphérique présente quatre rainures (b). Ces rainures se trouvent au milieu de chacun des bords dudit récipient. Le moyen de fermeture dudit conditionnement consiste en une plaque plane du type "couvercle" (d). La figure 1B présente le conditionnement décrit ci-dessus alors que le couvercle a été collés sur le récipient, il apparaît clairement que les rainures ainsi recouvertes donnent naissance à des orifices présentant une coupe de demi-cercle.

### II. Exemple d'une plaque de conditionnements utilisable dans la présente invention comportant des conditionnements sécables munis, chacun, d'un dispositif d'accrochage aux plantes.

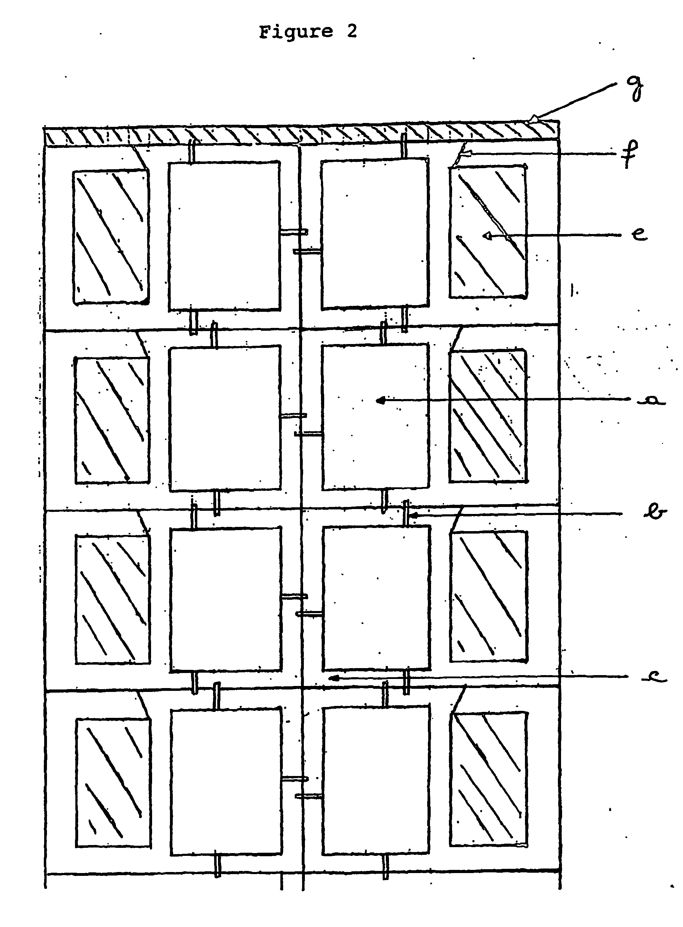

La figure 2 présente une plaque de conditionnements comportant deux séries de conditionnements tels que ceux définis à l'exemple 1 ci-dessus. Le conditionnement présenté dans la figure 2 diffère de celui présenté dans la figure 1 de par la présence de trois orifices de sortie (et non quatre) et de par la présence d'un système d'accrochage pour chaque conditionnement. Cette figure présente la disposition des rainures (ou orifices de sortie) d'un conditionnement par rapport aux conditionnements qui l'entourent.

Chaque rainure s'étend au-delà de la séparation de deux conditionnements de façon à ouvrir ledit orifice au moment où les conditionnements sont dissociés les uns des autres. On peut également noter que les rainures d'un conditionnement par rapport à un autre conditionnement adjacent sont disposées en quinconce de façon à ce que les orifices formés ne fassent pas communiquer les conditionnements les uns avec les autres. Cependant, dans le cadre de la présente invention, les orifices d'un conditionnement peuvent correspondre aux orifices d'un conditionnement adjacent et ce, de façon, à ce que lesdits orifices forment une voie de communication entre les conditionnements. Les deux formes de mise en oeuvre sont envisageables.

Le système d'accrochage est formé à partir de la prolongation sur un côté de la bande périphérique entourant le récipient, solidarisée avec un prolongement équivalent du couvercle fermant ledit récipient. Ces prolongations solidarisées au moyen d'un encollage permettent d'obtenir des structures solides qui peuvent être évidées (e) pour matérialiser le système d'accrochage du type crochet. Une ouverture latérale du crochet (f) peut également être nécessaire pour faciliter l'accrochage des conditionnements.

Enfin, une bande périphérique (g) de fermeture des conditionnements d'extrémité de la plaque permet de fermer les orifices latéraux (ou orifices extérieurs) des conditionnements qui sont normalement ouverts.

### III. Exemple d'une plaque de conditionnements utilisable dans la présente invention comportant des conditionnements sans dispositif d'accrochage aux plantes.

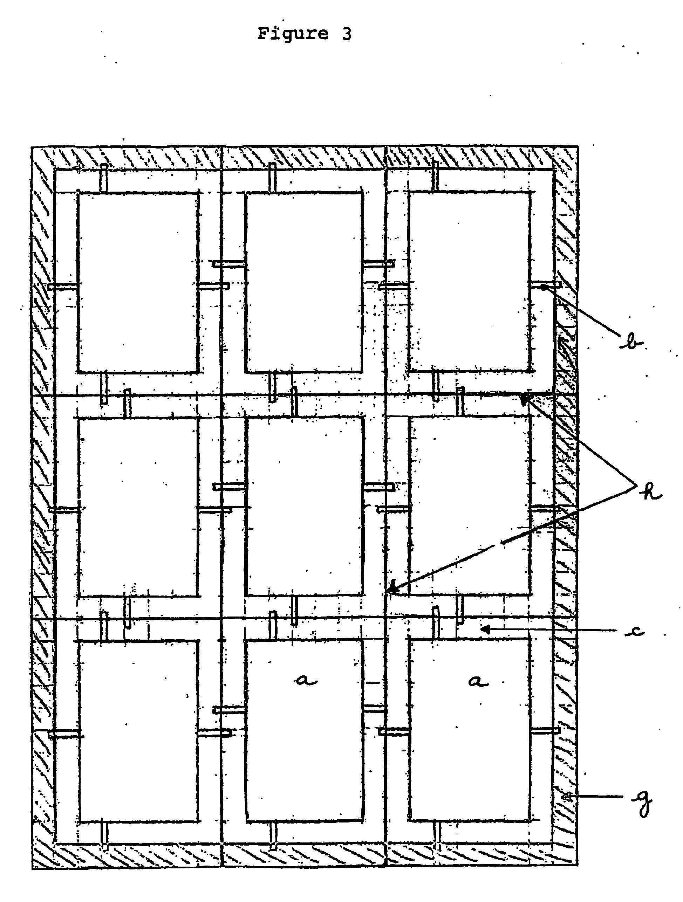

La figure 3 présente une plaque de conditionnements comportant trois rangées de trois conditionnements. Ces conditionnements sont strictement identiques au conditionnement décrit à l'exemple 1. En effet, chacun des conditionnements comprend un récipient (a), des rainures (b) engendrant chacune un orifice de sortie lorsque le moyen de fermeture est disposé sur chacun des récipients et une bande périphérique (c). Chacun des conditionnements présente quatre orifices de sortie et aucun dispositif d'accrochage aux plantes.

Cette plaque de conditionnements présente également une bande périphérique (g) de la plaque maintenant fermés les orifices extérieurs, telle que présentée à l'exemple 2.

Les différents conditionnements sont sécables et sont dissociés suivant des traits de pré-découpe (h). Les orifices de sortie présentent les mêmes propriétés que ceux décrits dans l'exemple 2 avec une distribution en quinconce et une longueur supérieure à La longueur de la bande périphérique entourant chacun des récipients.

### IV. Exemple d'une autre forme de conditionnement utilisable dans la présente invention et d'une plaque de conditionnements comportant de tels conditionnements.

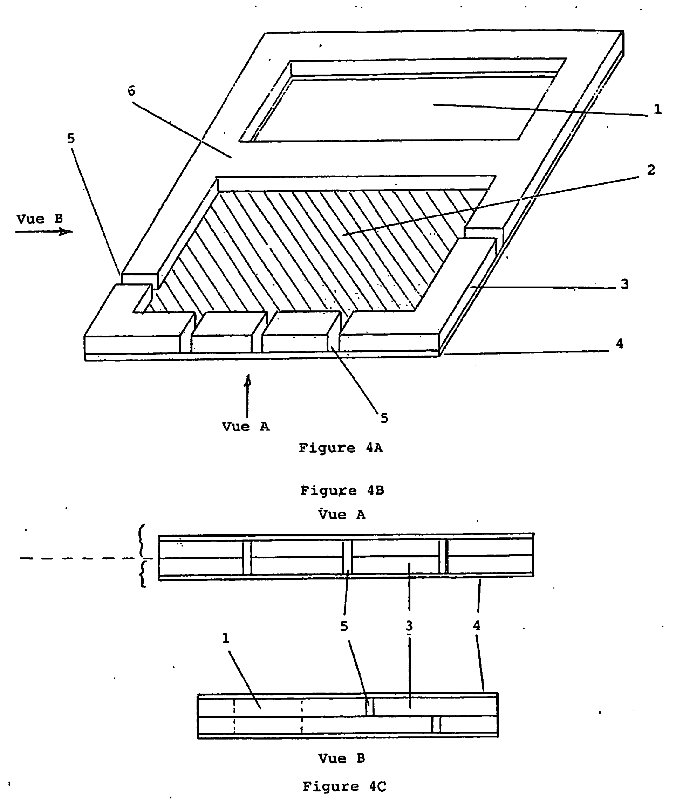

La figure 4 présente une autre forme de conditionnement utilisable dans la présente invention. La figure 4A présente plus particulièrement ledit conditionnement sans moyen de fermeture également appelé ci-après « demi-conditionnement ». Dans ce demi-conditionnement, le récipient est formé par une alvéole elle-même aménagée par évidage d'un carton de forte épaisseur (3) obturé et renforcé par un carton de faible épaisseur (4). Le carton de forte épaisseur (3) sera choisi en fonction de la hauteur de l'alvéole désirée ; ce carton (3) peut présenter, par exemple, une épaisseur de 1 à 3 mm pour des trichogrammes et plus pour des auxiliaires de plus grande taille. Les languettes telles que définies dans ce conditionnement présentent la forme d'une bande périphérique. Elles sont constituées parle carton de forte épaisseur (3) renforcé par le carton de faible épaisseur (4). Cette bande périphérique présente 5 rainures (5) obtenues par évidage du carton de forte épaisseur (3). Trois de ces rainures se trouvent sur la base inférieure du présent conditionnement et sont régulièrement réparties sur la longueur dudit conditionnement. Chaque côté du conditionnement comporte une rainure localisée, par exemple, au 1/3 et au 2/3 de la largeur de l'alvéole. Les rainures peuvent être disposées différemment, y compris à la moitié de la largeur de l'alvéole si l'on désire obtenir des alvéoles communicantes. Chacune des rainures présente des dimensions choisies par l'homme du métier en fonction de l'auxiliaire à conditionner, à titre d'exemple, le tableau 1 présente certaines des dimensions utilisables pour ces rainures. La surface hachurée correspond à la surface de l'alvéole (2) sur laquelle sont collés les stades immobiles des auxiliaires avec de la nourriture appropriée en fonction dudit auxiliaire. Le conditionnement présente également un crochet pour le fixer sur les végétaux (1) ; ce crochet est réalisé en effectuant un trou dans le carton de forte épaisseur (3) et dans le carton de faible épaisseur (4).

Le demi-conditionnement peut être fermé soit en collant sur la face encollée (6) un autre demi-conditionnement identique ou non, suivant la position des rainures de sortie, ce qui permet d'augmenter ou de réduire le nombre et la dimension des orifices de sortie.

Les figures et 4C représentent deux vues d'un conditionnement obtenu en collant ensemble deux demi-conditionnements identiques tels que ceux décrits à la figure 4A. La figure 4B correspond à une vue de la base du conditionnement ainsi obtenu. Sur la base, les rainures des demi-conditionnements correspondent deux par deux permettant d'obtenir une fente de sortie présentant une hauteur double par rapport à celle d'une rainure. La figure 4C correspond à une vue de côté du conditionnement ainsi obtenu. Dans ce cas, les rainures des demi-conditionnements ne sont pas continues, la fente de sortie ayant la hauteur d'une rainure. Le conditionnement ainsi obtenu présente donc 3 fentes de sortie sur sa base et 4 sur ses côtés.

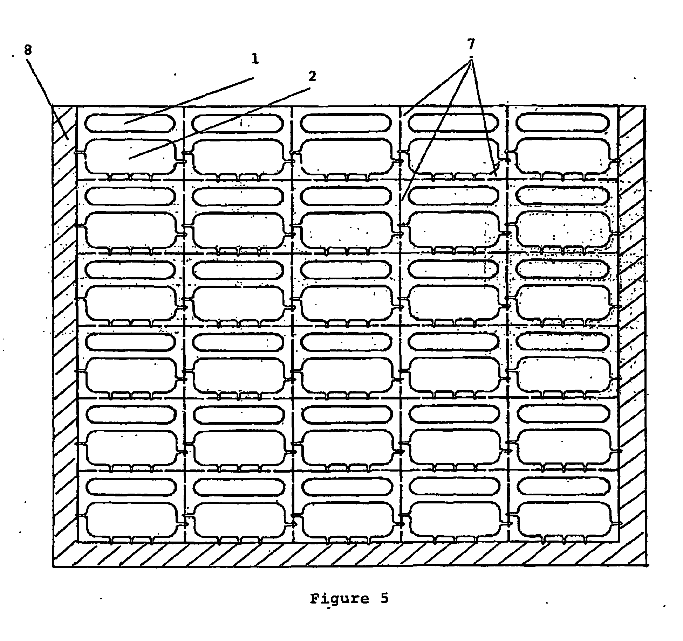

La figure 5 présente une plaque de conditionnements sécables comportant 30 conditionnements tels que ceux décrits à la figure 4. Il est clair que l'homme du métier saura adapter suivant les besoins le nombre de conditionnements par plaques. Cette plaque de conditionnements comprend 5 conditionnements placés côte à côte et ce, sur 6 rangées. Les conditionnements sont sécables et sont dissociés suivant des traits de pré-découpe (7) présentant des points de fixation. Les trous de sortie pour un conditionnement donné dans cette plaque sont obturés tant que les conditionnements ne sont pas dissociés. En effet, les trous de sortie de la base du conditionnement N° X de la rangée Y sont maintenus fermés par le bord du crochet (1) du conditionnement N° X de la rangée Y+1. Compte-tenu de la répartition des rainures sur les côtés de conditionnements adjacents, chacune des rainures est obturée par le conditionnement adjacent. Une bande périphérique (8) permet d'obstruer les trous de sortie des conditionnements qui ont un côté latéral et/ou une base non en contact avec un autre conditionnement.

## Revendications

1. Utilisation d'un conditionnement pour protéger et libérer des insectes et/ou acariens auxiliaires dans le cadre de la lutte biologique, ledit conditionnement comprenant :
- un récipient (a) présentant au moins une languette (c), elle-même présentant au moins une rainure (b) et
- un moyen (d) pour fermer ledit récipient (a), ledit moyen (d) de fermeture étant disposé sur ledit récipient (a), ladite rainure (b) et ledit moyen (d) pour fermer ledit récipient forment un orifice permettant la sortie desdits insectes et/ou acariens.

2. Utilisation selon la revendication 1, dans laquelle ledit récipient (a) se présente sous la forme d'une demi-sphère, d'une capsule, d'une boite ou d'une alvéole.

3. Utilisation selon l'une quelconque des revendications 1 ou 2, dans laquelle ledit conditionnement comprend plusieurs languettes (c) présentant chacune au moins une rainure (b).

4. Utilisation selon la revendication 3, dans laquelle lesdites languettes (c) forment une bande périphérique à la surface du récipient.

5. Utilisation selon l'une quelconque des revendications 1 à 4, dans laquelle ledit moyen (d) de fermeture du récipient est choisi parmi un deuxième récipient ou une surface plane.

6. Utilisation selon l'une quelconque des revendications 1 à 5, dans laquelle ledit moyen (d) de fermeture du récipient comporte des micro-perforations.

7. Utilisation selon l'une quelconque des revendications 1 à 6, dans laquelle le récipient (a), la (ou les) languette(s) (c) et le moyen (d) de fermeture dudit récipient sont dans un matériau identique ou différent choisi parmi le carton, la cellulose moulée et le plastique.

8. Utilisation selon l'une quelconque des revendications 1 à 7, dans laquelle ledit conditionnement présente un moyen d'accrochage.

9. Utilisation selon l'une quelconque des revendications 1 à 8, dans laquelle lesdits insectes et/ou acariens auxiliaires sont conditionnés sous forme mobile ou non et sont libérés sous forme mobile larvaire ou adulte.

10. Utilisation selon l'une quelconque des revendications 1 à 9, dans laquelle lesdits insectes et/ou acariens auxiliaires sont avantageusement choisis dans le groupe constitué par les trichogrammes, les punaises prédatrices, les acariens prédateurs et les hyménoptères parasites de pucerons.

11. Utilisation selon l'une quelconque des revendications 1 à 10, dans laquelle ledit conditionnement se trouve dans une plaque comprenant au moins deux conditionnements.

12. Utilisation selon la revendication 11, **caractérisée en ce que** lesdits conditionnements sont des conditionnements jumelés et sécables.

13. Utilisation selon la revendication 12, dans laquelle la plaque comprend une bande périphérique de fermeture adaptée pour fermer les orifices disposés aux extrémités de la plaque.

14. Utilisation selon l'une des revendications 12 ou 13, dans laquelle les rainures des conditionnements sont disposées de manière que les orifices formés ne fassent pas communiquer lesdits conditionnements les uns avec les autres.

## Claims

1. The use of a packaging for protecting and releasing auxiliary insects and/or mites within the scope of biological control, said packaging comprising:
- a container (a) having at least one tab (c) itself having at least one groove (b) and
- a means (d) for closing said packaging (a), said closing means (d) being positioned on said container (a), said groove (b) and said means (d) for closing said container forming an orifice allowing the exit of said insects and/or mites.

2. The use according to claim 1, wherein said container (a) appears in the form of a half-sphere, a capsule, a box or a cell.

3. The use according to any of claims 1 to 2, wherein said packaging comprises several tabs (c) each having at least one groove (b).

4. The use according to claim 3, wherein said tabs (c) form a peripheral band at the surface of the container.

5. The use according to any of claims 1 to 4, wherein said means (d) for closing the container is selected from a second container or a planar surface.

6. The use according to any of claims 1 to 5, wherein said means (d) for closing the container includes micro-perforations.

7. The use according to any of claims 1 to 6, wherein the container (a), the tab(s) (c) and the means (d) for closing said container are in an identical or different material selected from cardboard, molded cellulose and plastic.

8. The use according to any of claims 1 to 7, wherein said packaging has a hooking-up means.

9. The use according to any of claims 1 to 8, wherein said auxiliary insects and/or mites are either conditioned in a mobile form or not and are released in a larval or adult mobile form.

10. The use according to any of claims 1 to 9, wherein said auxiliary insects and/or mites are advantageously selected from the group formed by trichogrammas, predatory bugs, predatory mites and hymenoptera parasites of aphids.

11. The use according to any of claims 1 to 10, wherein said packaging is found in a plate comprising at least two packagings.

12. The use according to claim 11, **characterized in that** said packagings are twin and severable packagings.

13. The use according to claim 12, wherein the plate comprises a peripheral closing strip adapted for closing the orifices positioned at the ends of the plate.

14. The use according to any of claims 12 or 13, wherein the grooves of the packagings are positioned so that the formed orifices do not provide communication of said packagings with each other.

## Patentansprüche

1. Verwendung einer Verpackung zum Schutz und zur Freilassung helfender Insekten und/oder Milben im Rahmen der biologischen Bekämpfung, wobei die Verpackung umfasst:
- einen Behälter (a), der mindestens eine Feder (c) aufweist, die selbst mindestens eine Rille (b) aufweiset, und
- ein Mittel (d) zum Verschließen des Behälters (a), wobei das Verschlussmittel (d) auf dem Behälter (a) angeordnet ist, wobei die Rille (b) und das Mittel (d) zum Verschließen des Behälters eine Öffnung bilden, die den Austritt der Insekten und/oder Milben erlaubt.

2. Verwendung nach Anspruch 1, wobei der Behälter (a) die Form einer Halbkugel, einer Kapsel, einer Schachtel oder einer Wabe hat.

3. Verwendung nach einem der Ansprüche 1 oder 2, wobei die Verpackung mehrere Federn (c) umfasst, von denen jede mindestens eine Rille (b) aufweist.

4. Verwendung nach Anspruch 3, wobei die Federn (c) einen Umfangsstreifen auf der Fläche des Behälters bilden.

5. Verwendung nach einem der Ansprüche 1 bis 4, wobei das Verschlussmittel (d) des Behälters aus einem zweiten Behälter oder einer ebenen Fläche ausgewählt ist.

6. Verwendung nach einem der Ansprüche 1 bis 5, wobei das Verschlussmittel (d) des Behälters' Mikroperforationen umfasst.

7. Verwendung nach einem der Ansprüche 1 bis 6, wobei der Behälter (a), die Feder(n) (c) und das Verschlussmittel (d) des Behälters aus einem identischen oder unterschiedlichen Material sind, das aus Pappe, Faserpressstoff und Kunststoff ausgewählt ist.

8. Verwendung nach einem der Ansprüche 1 bis 7, wobei die Verpackung ein Befestigungsmittel aufweist.

9. Verwendung nach einem der Ansprüche 1 bis 8, wobei die helfenden Insekten und/oder Milben in beweglicher Form oder nicht verpackt sind und in beweglicher Form als Larve oder erwachsen freigelassen werden.

10. Verwendung nach einem der Ansprüche 1 bis 9, wobei die helfenden Insekten und/oder Milben in vorteilhafter Weise aus der Gruppe ausgewählt sind, die von den Trichogramma, den Raubwanzen, den Raubmilben und den parasitischen Laus-Hymenopteren gebildet wird.

11. Verwendung nach einem der Ansprüche 1 bis 10, wobei sich die Verpackung in einer Platte befindet, die mindestens zwei Verpackungen umfasst.

12. Verwendung nach Anspruch 11, **dadurch gekennzeichnet, dass** die Verpackungen Zwillings- und untrennbare Verpackungen sind.

13. Verwendung nach Anspruch 12, wobei die Platte einen Verschluss-Umfangsstreifen umfasst, der geeignet ist, die an den Enden der Platte angeordneten Öffnungen zu verschließen.

14. Verwendung nach einem der Ansprüche 12 oder 13, wobei die Verpackungsrillen derart angeordnet sind, dass die gebildeten Öffnungen keine Kommunikation der Verpackungen untereinander bewirken.
